(19) Europäisches Patentamt
European Patent Office
Office européen des brevets

(11) EP 1 635 833 B1

(12) **EUROPEAN PATENT SPECIFICATION**

(45) Date of publication and mention of the grant of the patent:
**14.05.2008 Bulletin 2008/20**

(21) Application number: **04741745.6**

(22) Date of filing: **07.06.2004**

(51) Int Cl.:
*A61K 31/496* (2006.01) *A61P 25/04* (2006.01)
*A61K 31/485* (2006.01) *A61K 31/4468* (2006.01)

(86) International application number:
**PCT/EP2004/051050**

(87) International publication number:
**WO 2004/110451 (23.12.2004 Gazette 2004/52)**

(54) **COMBINATION OF OPIOIDS AND A PIPERAZINE DERIVATIVE FOR THE TREATMENT OF PAIN**

KOMBINATION VON OPIOIDEN UND EINEM PIPERAZIN-DERIVAT FÜR DIE BEHANDLUNG VON SCHMERZEN

ASSOCIATION A BASE D'OPIOIDES ET D'UN DERIVE DE PIPERAZINE DESTINEE AU TRAITEMENT DE LA DOULEUR

(84) Designated Contracting States:
**AT BE BG CH CY CZ DE DK EE ES FI FR GB GR HU IE IT LI LU MC NL PL PT RO SE SI SK TR**

(30) Priority: **10.06.2003 WOPCT/EP03/06118**

(43) Date of publication of application:
**22.03.2006 Bulletin 2006/12**

(73) Proprietor: **Janssen Pharmaceutica NV**
**2340 Beerse (BE)**

(72) Inventors:
- **JANSSENS, Frans Eduard,**
**Janssen Pharmaceutica N.V.**
**B-2460 Beerse (BE)**
- **SOMMEN, François Maria,**
**Janssen Pharmaceutica N.V.**
**B-2340 Beerse (BE)**
- **LEENAERTS, Joseph E.,**
**Janssen Pharmaceutica N.V.**
**B-2340 Beerse (BE)**
- **VAN ROOSBROECK, Y.E.M.,**
**Janssen Pharmaceutica N.V.**
**B-2340 Beerse (BE)**
- **MEERT, Theo Frans,**
**Janssen Pharmaceutica N.V.**
**B-2340 Beerse (BE)**

(74) Representative: **Daelemans, Frank F.R. et al**
**Janssen Pharmaceutica N.V.**
**J&J Patent Law Department**
**Turnhoutseweg 30**
**2340 Beerse (BE)**

(56) References cited:
**WO-A-01/30371** **WO-A-02/32867**
**WO-A-96/20009** **WO-A-97/16440**
**WO-A-03/066621** **GB-A- 2 287 404**

- **BOUNTRA C ET AL: "ANTI-EMETIC PROFILE OF A NON-PEPTIDE NEUROKININ NK1 RECEPTOR ANTAGONIST, CP-99,994, IN FERRETS" EUROPEAN JOURNAL OF PHARMACOLOGY, AMSTERDAM, NL, vol. 249, 1993, pages R03-R04, XP000571021 ISSN: 0014-2999**
- **TATTERSALL F D ET AL: "ENANTIOSELECTIVE INHIBITION OF APOMORPHINE-INDUCED EMESIS IN THE FERRET BY THE NEUROKININ1 RECEPTOR ANTAGONIST CP-99,994" NEUROPHARMACOLOGY, PERGAMON PRESS, OXFORD, GB, vol. 33, no. 2, 9 May 1996 (1996-05-09), pages 259-260, XP000570654 ISSN: 0028-3908**
- **GARDNER, C. J. ET AL: "GR 205171: A novel antagonist with high affinity for the tachykinin NK1 receptor, and potent broad-spectrum anti-emetic activity" REGULATORY PEPTIDES ,65(1), 45-53 CODEN: REPPDY; ISSN: 0167-0115, 1996, XP002297280**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 635 833 B1

## Description

### Field of the Invention

This invention concerns novel formulations for opioid-based treatments of pain and/or nociception comprising opioid analgesics and 1-(1,2-disubstituted piperidinyl)-4-substituted piperazine derivatives having neurokinin antagonistic activity, in particular $NK_1$ antagonistic activity, the use of said formulation for the manufacture of a medicament for the prevention and/or treatment of emesis, in particular nausea and vomiting, pain and/or nociception, in particular in opioid-based acute and chronic pain treatments, more in particular in inflammatory, post-operative, emergency room (ER), breakthrough, neuropathic and cancer pain treatments and the use of an $NK_1$-receptor antagonist for the manufacture of a medicament for the prevention and/or treatment of respiratory depression and tolerance in opioid-based treatments of pain.

### Background of The Invention

Opioid analgesics are the cornerstone of pain treatment, especially in the segment of moderate to severe acute and chronic pain. However, side-effects such as nausea/vomiting, constipation, respiratory depression and tolerance limit their use. The lowering of the high incidence of nausea and vomiting with many clinically used opioids is particularly considered as a major unmet medical need.

Neurokinins belong to a family of short peptides that are widely distributed in the mammalian central and peripheral nervous system (Bertrand and Geppetti, Trends Pharmacol. Sci. 17:255-259 (1996) ; Lundberg, Can. J. Physiol. Pharmacol. 73:908-914 (1995) ; Maggi, Gen. Pharmacol 26:911-944 (1995); Regoli et al., Pharmacal. Rev. 46 (1994)). They share the common C-terminal sequence Phe-Xaa-Gly-Leu-Met-$NH_2$. Neurokinins released from peripheral sensory nerve endings are believed to be involved in neurogenic inflammation. In the spinal cord/central nervous system, neurokinins may play a role in pain transmission/perception and in some autonomic reflexes and behaviors. The three major neurokinins are Substance P (SP), Neurokinin A ($NK_A$) and Neurokinin B ($NK_B$) with preferential affinity for three distinct receptor subtypes, termed $NK_1$, $NK_2$, and $NK_3$, respectively. However, functional studies on cloned receptors suggest strong functional cross-interaction between the 3 neurokinins and their corresponding receptors (Maggi and Schwartz, Trends Pharmacol. Sci. 18: 351-355 (1997)). Species differences in structure of $NK_1$ receptors are responsible for species-related potency differences of $NK_1$ antagonists (Maggi, Gen. Pharmacol. 26:911-944 (1995) ; Regoli et al., Pharmacal. Rev. 46(4):551-599 (1994)). The human $NK_1$ receptor closely resembles the $NK_1$ receptor of guinea-pigs and gerbils but differs markedly from the $NK_1$ receptor of rodents. The development of neurokinin antagonists has led to date to a series of peptide compounds of which might be anticipated that they are metabolically too labile to be employed as pharmaceutically active substances (Longmore J. et al., DN&P 8(1):5-23 (1995)). $NK_1$-antagonists have been studied for a wide variety of indications including emesis, (stress-related) anxiety states, inflammatory responses, smooth muscle contraction and pain perception. $NK_1$-antagonists are in development for indications such as emesis, anxiety and depression, irritable bowel syndrome (IBS), circadian rhythm disturbances, visceral pain, neurogenic inflammation, asthma, micturition disorders, pancreatitis and nociception.

It has now surprisingly been found that a particular class of compounds with predominantly $NK_1$-activity reduces to a large extent a number of unwanted side-effects associated with opioid analgesics, thereby increasing the total tolerability of said opioids in pain treatment, in particular in opioid-based acute and chronic pain treatments, more in particular in inflammatory, post-operative, emergency room (ER), breakthrough, neuropathic and cancer pain treatments. More specifically, it was found in opioid-based treatments of pain that emesis was inhibited, respiratory depression was reduced, the tolerance for opioids was prevented and constipation was not worsened. Also, due to the intrinsic antinociceptive activity of $NK_1$-antagonists, even some increase in opioid efficacy is noted, thereby creating the option to reduce the opioid dose without effecting its analgesic action. Finally, by this combination, psychotropic properties were added to the analgesic efficacy by reducing stress, anxiety and depression.

### Background prior art

1-(1,2-disubstituted piperidinyl)-4-substituted piperazine derivatives were disclosed in WO 97/16440-A1, published May 9,1997 by Janssen Pharmaceutica N.V. for use as substance P antagonists.

Formulations containing $NK_1$-antagonists and opioid analgesics for the prevention and/or treatment of pain and/or nociception are disclosed in WO 96/20009 (Merck, July 4, 1996) and WO 97/25988 (Eli Lilly, July 24, 1997). There is no mentioning of the reduction of side-effects apart from emesis.

GB 2 287 disclose combination of opioid analgesic and tachykinin antagonist in the treatment of pain. WO 01/30371 disclose chimeric compounds that bind to both opioid receptors and the NK1 receptor produce effective analgesia and inhibit the development of tolerance. WO 02/32867 disclose piperidinyl-piperazines as neurokynin (e.g.

NK1) antagonists counter the emetic effects of opioid analgesics like morphine. Bountra et al. European Journal of Pharmacology, Vol. 249, (1993), R03-R04, and Tattersall et al. Neuropharmacology, Vol. 33, no. 2, (1996), 259-260, disclose CP 99,994, an NK1 antagonist.

Description of the Invention

The present invention relates to a pharmaceutical composition comprising a pharmaceutically acceptable carrier and, as active ingredients, a therapeutically effective amount of an opioid analgesic and compound 27 (+)-(B)-*trans*-4-[1-[3,5-bis(trifluoromethyl)benzoyl]-2-(phenylmethyl)-4-piperidinyl]-N-(2,6-dimethylphenyl)-1-piperazine acetamide, the pharmaceutically acceptable acid or base addition salts thereof, the *N*-oxide form thereof and the prodrugs thereof.
Preferred embodiments are described in dependent claims 2-6.
Most in particular, the pharmaceutical composition comprises a compound selected from the group of :

- (+)-(B)-*trans*-4-[1-[3,5-bis(trifluoromethyl)benzoyl]-2-(phenylmethyl)-4-piperidinyl]-*N*-(2,6-dimethylphenyl)-1-piperazine acetamide; compound 27

- (+)-(B)-*trans*-4-[1-[3,5-bis(trifluoromethyl)benzoyl]-2-(phenylmethyl)-4-piperidinyl]-*N*-(2,6-dimethylphmyl)-1-piperazine acetamide, (L)-malic acid (1:1). compound 95

The pharmaceutically acceptable salts are defined to comprise the therapeutically active non-toxic acid addition salts forms that the compounds 95, 27 are able to form. Said salts can be obtained by treating the base form of the compounds 95, 27 with appropriate acids, for example inorganic acids, for example hydrohalic acid, in particular hydrochloric acid, hydrobromic acid, sulfuric acid, nitric acid and phosphoric acid; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid and pamoic acid.
The compounds 95, 27 containing acidic protons may also be converted into their therapeutically active non-toxic metal or amine addition salts forms by treatment with appropriate organic and inorganic bases. Appropriate base salts forms comprise, for example, the ammonium salts, the alkaline and earth alkaline metal salts, in particular lithium, sodium, potassium, magnesium and calcium salts, salts with organic bases, e.g. the benzathine, *N*-methyl-D-glucamine, hybramine salts, and salts with amino acids, for example arginine and lysine.
Conversely, said salt forms can be converted into the free forms by treatment with an appropriate base or acid.
The term addition salt as used in the framework of this application also comprises the solvates that the compounds according to Formula (I) as well as the salts thereof, are able to form. Such solvates are, for example, hydrates and alcoholates.
The *N*-oxide forms of compounds 95, 27 are meant to comprise those compounds wherein one or several nitrogen atoms are oxidized to the so-called *N*-oxide, particularly those *N*-oxides wherein one or more tertiary nitrogens (e.g of the piperazinyl or piperidinyl radical) are *N*-oxidized. Such *N*-oxides can easily be obtained by a skilled person without any inventive skills and they are obvious alternatives for the compounds since these compounds are metabolites, which are formed by oxidation in the human body upon uptake As is generally known, oxidation is normally the first step involved in drug metabolism (Textbook of Organic Medicinal and Pharmaceutical Chemistry, 1977, pages 70- 75). As is also generally known, the metabolite form of a compound can also be administered to a human instead of the compound per se, with much the same effects.
compounds 95, 27 possess at least 2 oxydizable nitrogens (tertiary amines moieties). It is therefore highly likely that *N*-oxides are to form in the human metabolism.
compounds 95, 27 may be converted to the corresponding *N*-oxide forms following art-known procedures for converting a trivalent nitrogen into its *N*-oxide form. Said *N*-oxidation reaction may generally be carried out by reacting the starting material with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert*-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.
Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Compounds encompassing double

bonds can have an E or Z-stereochemistry at said double bond.

Following CAS nomenclature conventions, when two stereogenic centers of known absolute configuration are present in a molecule, an R or S descriptor is assigned (based on Cahn-Ingold-Prelog sequence rule) to the lowest-numbered chiral center, the reference center. The configuration of the second stereogenic center is indicated using relative descriptors [*R**,*R**] or *[R*,S*]*, where R* is always specified as the reference center and [*R*,R**] indicates centers with the same chirality and [*R*,S**] indicates centers of unlike chirality. For example, if the lowest-numbered chiral center in the molecule has an *S* configuration and the second center is R, the stereo descriptor would be specified as *S*-[*R**, *S**]. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system (hydrogen atom in compounds 95, 27 relative to the position of the highest priority substituent on the reference atom is denominated "α", if it is on the same side of the mean plane determined by the ring system, or "β", if it is on the other side of the mean plane determined by the ring system.

Compounds 27, 95, and some of the intermediate compounds have at least two stereogenic centers in their structure.

The invention also comprises pharmaceutical compositions according to the invention comprising derivative compounds (usually called "pro-drugs") of the pharmacologically-active compounds according to the invention, which are degraded *in vivo* to yield the compounds according to the invention. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985, 29, pp. 455-473.

Pro-drugs forms of the pharmacologically-active compounds according to the invention will generally be compound 27 or 95 the pharmaceutically acceptable acid or base addition salts thereof, the stereochemically isomeric forms thereof and the *N*-oxide form thereof having an acid group which is esterified or amidated. Included in such esterified acid groups are groups of the formula -COOR$^x$, where R$^x$ is a $C_{1-6}$alkyl, phenyl, benzyl or one of the following groups :

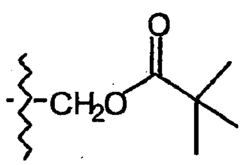
,
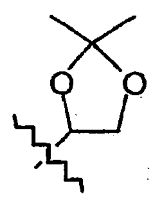

Amidated groups include groups of the formula - CONR$^y$R$^z$, wherein R$^y$ is H, $C_{1-6}$alkyl, phenyl or benzyl and R$^z$ is -OH, H, $C_{1-6}$alkyl, phenyl or benzyl. Compounds according to the invention having an amino group may be derivatised with a ketone or an aldehyde such as formaldehyde to form a Mannich base. This base will hydrolyze with first order kinetics in aqueous solution.

The compound 27 or 95 is prepared in the processes described below may be synthesized in the form of racemic mixtures of enantiomers that can be separated from one another following art-known resolution procedures. The racemic compounds of 95 or 27 may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

In the framework of this application, the term opioid means opium-like or morphine-like in terms of pharmacological action. The broad group of opium alkaloids, synthetic derivatives related to the opium alkaloids, and the many naturally occuring and synthetic peptides with morphine-like pharmacological effects is called opioids. In addition to having pharmacological effects similar to those of morphine, a compound must be antagonized by an opioid antagonist such as naloxone to be classified as an opioid. The neuronally located proteins to which opioid agents bind to initiate a biological response are called opioid receptors. Opioids can act peripherally and centrally.

Suitable opioids or opioid analgesics for use in the present invention include one or more compounds selected from the group of alfentanil, buprenorphine, butorphanol, carfentanil, codeine, diacetylmorphine, dihydrocodeine, fentanyl, hydrocodone, hydromorphone, levorphanol, lofentanil, meperidine, methadone, morphine, nalbuphine, oxycodone, oxymorphone, pentazocine, propoxyphene, remifentanil and sufentanil; and pharmaceutical acceptable salts and derivatives thereof.

Because of their widespread use as analgesics, preferred opioid analgesics of use in the present invention are one or more compounds selected from the group of oxycodone, codeine, morphine, fentanyl, buprenorphine, hydrocodone, hydromorphone and pharmaceutical acceptable salts and derivatives thereof.

Suitable pharmaceutically acceptable salts of the opioid analgesics of use in the present invention include those salts described above in relation to the salts of the $NK_1$-antagonist.

Preferred salts of opioid analgesics of use in the present invention include alfentanil hydrochloride, buprenorphine hydrochloride, butorphanol tartrate, codeine phosphate, codeine sulphate, diacetylmorphine hydrochloride, dihydrocodeine bitartrate, fentanyl citrate, hydrocodone bitartrate, hydromorphone hydrochloride, levorphanol tartrate, meperidine hydrochloride, methadone hydrochloride, morphine sulphate, morphine hydochloride, morphine tartrate, nalbuphine hydrochloride, oxymorphone hydrochloride, pentazocine hydrochloride, propoxyphene hydrochloride and propoxyphene napsylate (2-naphthalene sulphonic acid (1:1) monohydrate).

More particular preferred opioid analgesics of use in the present invention are morphine sulphate and fentanyl citrate.

Pharmacology

compound 27 or 95 are potent inhibitors of neurokinin-mediated effects, in particular those mediated via the $NK_1$ receptor, and may therefore be described as neurokinin antagonists, especially as substance P antagonists, as indicated *in vitro* by the antagonism of substance P-induced relaxation of pig coronary arteries which is described hereinafter. The binding affinity of the present compounds for the human, guinea-pig and gerbil neurokinin receptors may be determined *in vitro* in a receptor binding test using $^3$H-substance-P as radioligand. The subject compounds also show substance-P antagonistic activity *in vivo* as may be evidenced by, for instance, the antagonism of substance P-induced plasma extravasation in guinea-pigs, or the antagonism of drug-induced emesis in ferrets (Watson et al., Br. J. Pharmacol. 115:84-94 (1995)).

The combination of an opioid analgesic with an $NK_1$ antagonist results in improved efficacy. Additional to the gain in efficacy, this combination also reduces several of the side-effects currently present with clinically used opioids. $NK_1$ receptor antagonists potentiating the analgesic activity of opioids require lower doses, resulting in a reduced risk of opioid side-effects, in particular emesis, respiratory depression and tolerance. But additionally it's seen that at similar doses (not lower opioid doses) there are also benefits of adding NK1 to opioid.

Respiratory depression is the most serious side effect of opioid analgesics and is the primal cause of death from overdose. Opioids decrease the sensitivity of chemoreceptors in the brainstem to carbon dioxide, a normal stimulus of ventilatory reflexes. The result is a blunting of the ventilatory response to increases in the carbon dioxide tension ($P_{CO_2}$) in blood and cerebrospinal fluid. At equally effective analgesic doses, most opioids produce a similar degree of respiratory depression, as shown by an elevation in the blood $P_{CO_2}$. This effect is at least additive to that produced by other drugs that depress CNS functions, including general anesthetics and sedative-hypnotics. The mild respiratory depression produced by therapeutic doses of opioids is normally of little consequence. However, opioid analgesics must be used cautiously in patients with traumatic head injuries, with emphysema and who are morbidly obese.

At three to five times its usual analgesic dose, morphine can cause respiratory arrest in the nontolerant patient. In contrast, much higher doses will have minimal respiratory effects in morphine-tolerant individuals.

Tolerance refers to a reduced drug effect with repeated use and/or a need for higher doses to produce the same effect. Because tolerance does not occur to the same extent for all effects, drug abusers who take increasing amounts of drugs risk exposure to those effects to which tolerance does not develop. Tolerance develops to many of the effects of opioids. With repeated drug administration, larger doses are necessary to produce the same pharmacological response. The rate of tolerance development varies with the affected tissue of organ. Tolerance develops rapidly to the antiemetic effects of opioids; more gradually to their analgesic, endocrine and respiratory depressant effects ; and virtually not at all to their constipating and miotic effects.

The compounds according to the invention have shown to reduce unwanted side-effects induced by opioids. Such reduction can be tested by *in vivo* testing using several species (e.g. ferrets, gerbils, rats, guinea pigs) and several pain models, covering pain models aiming at different states of acute and chronic pain, as well as animal models aiming to profile opioid side effects (such as opioid-induced emesis, GI transit and respiratory depression). For instance, the compounds of the present invention :

- were able to inhibit the opioid-induced emesis in several species;

- did not reduce the antinociceptive properties of opioids in models of acute, visceral and high intensity pain;
- had an additive effect on the antinociceptive properties of opioids in models of inflammatory and chronic neuropathic pain;
- reduced the respiratory depression induced by opioids in several species;
- were able to reduce and overcome the tolerance observed with opioids daily administered in a model of chronic neuropathic pain;
- did not interfere with the discriminative central narcotic effects of opioids;
- had no effect on the pharmacokinetics of opioids when administered concomitantly. This excludes pharmacokinetic interactions as the origin of the pharmacological effects observed.

The present invention therefore also relates to the use of a pharmaceutical composition according to the invention for the manufacture of a medicament for the prevention and/or treatment of pain and/or nociception.

In particular, the present invention relates to the use of a pharmaceutical composition according to the invention for the manufacture of a medicament for the opioid-based prevention and/or treatment of acute and chronic pain, more in particular in inflammatory, post-operative, emergency room (ER), breakthrough, neuropathic and cancer pain treatments.

The present invention further relates to the use of a pharmaceutical composition according to the invention for the manufacture of a medicament for the prevention and/or treatment of emesis in opioid-based treatments of pain.

The present invention further relates to the use of a pharmaceutical composition according to the invention for the manufacture of a medicament for the prevention and/or treatment of nausea and vomiting in opioid-based treatments of pain.

The present invention also relates to the use of an $NK_1$-receptor antagonist, in particular the $NK_1$-receptor antagonist 27 or 95, the pharmaceutically acceptable acid or base addition salts thereof, the *N*-oxide form thereof and prodrugs thereof, for the manufacture of a medicament for the prevention and/or treatment of respiratory depression in opioid-based treatments of pain.

The present invention also relates to the use of an $NK_1$-receptor antagonist, in particular the $NK_1$-receptor antagonist 27 or 95, the pharmaceutically acceptable acid or base addition salts thereof, the stereochemically isomeric forms thereof, the *N*-oxide form thereof and prodrugs thereof, for the manufacture of a medicament for reducing and/or overcoming the tolerance observed with opioids, e.g. when daily administered in chronic neuropathic pain.

To prepare the pharmaceutical compositions of this invention, an effective amount of the active ingredient, optionally in addition salt form, is combined in intimate admixture with a pharmaceutically acceptable carrier, which carrier may take a wide variety of forms depending on the form of preparation desired for administration. The pharmaceutical compositions are desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment. Other compositions may be compositions in a form suitable for sublingual, intranasal or pulmonary application or suitable as eye droplets.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

Since the compounds according to the invention are potent orally administrable $NK_1$ antagonists, pharmaceutical compositions comprising said compounds for administration orally are especially advantageous.

The $NK_1$-receptor antagonist and the opioid analgesic may be formulated in a single pharmaceutical composition or alternatively in individual pharmaceutical compositions for simultaneous, separate or sequential use in accordance with the present invention. The pharmaceutical composition may also be a product comprising the $NK_1$- receptor antagonist and the opioid analgesic as separate unit dosages.

When administered in combination, either as a single or as separate pharmaceutical composition(s), the $NK_1$-receptor antagonist and the opioid analgesic are presented in a ratio which is consistent with the manifestation of the desired effect. In particular, the ratio by weight of the $NK_1$-antagonist to the opioid analgesic will suitably be approximately 1 to 1. Preferably, this ratio will be between 0.001 to 1 and 1000 to 1, and especially between 0.01 to 1 and 100 to 1.

A suitable dosage level for the $NK_1$-receptor antagonist is about 0.001 to 25 mg/kg per day, preferably about 0.005 to 10 mg/kg per day, and especially about 0.005 to 5 mg/kg day. The compounds may be administered on a regimen of up to 6 times per day, preferably 1 to 4 times per day.

The opioid analgesic may be administered at a dosage level up to conventional dosage levels for such analgesics, but preferably at a reduced level in accordance with the present invention. Suitable dosage levels will depend upon the analgesic effect of the chosen opioid analgesic, but typically suitable levels will be about 0.001 to 25 mg/kg per day, preferably 0.005 to 10 mg/kg per day, and especially 0.005 to 5 mg/kg day. The compound may be administered on a regimen of up to 6 times per day, preferably 1 to 4 times per day.

It will be appreciated that the amount of an $NK_1$-receptor antagonist and an opioid analgesic required for use in the prevention and/or treatment of pain and nociception will vary not only with the particular compounds or compositions selected but also with the route of administration, the nature of the condition being treated, and the age and condition of the human in need of such a treatment, and will ultimately be at the discretion of the attendant physician.

## Chemistry

The compounds according to the invention can generally be prepared by a succession of steps, each of which is known to the skilled person.

The preparation of these compounds and their intermediates is also described in WO 97/16440-A1, published May 9, 1997 by Janssen Pharmaceutica N.V, which is disclosed herein by reference as well as in other publications mentioned in WO 97/16440-A1, such as, e.g. EP-0,532,456-A.

The following examples are intended to illustrate and not to limit the scope of the present invention.

## Experimental Section

Hereinafter "RT" means room temperature, "THF" means tetrahydrofuran, "DIPE" means diisopropylether, "DCM" means dichloromethane and "DMF" means *N,N*-dimethylformamide.

## A. Preparation of the intermediate compounds

### Example A.2

a) A mixture of (±)-1,1-dimethyl 7-(phenylmethyl)-1,4-dioxa-8-azaspiro[4,5]decane-8-carboxylate (33.34 g; prepared according to the method described in EP-A-532,456) in HCl (6 N; 250 ml) was stirred at 70 °C for 1 hour and 30 minutes. The mixture was cooled, alkalized with NaOH while cooling to 25 °C, and extracted with DCM (100 ml). The organic layer was separated and the aqueous layer was extracted with $CH_2Cl_2$. Triethylamine (20.2 g), followed by 3,5-bis(trifluoromethyl)benzoyl chloride (27.7 g) dissolved in a little DCM were added and the mixture was stirred for 2 hours. The mixture was extracted with water, and the layers were separated. The organic layer was dried, filtered and the solvent evaporated. The residue was crystallized from DIPE, the precipitate was filtered off and dried, yielding 18.34 g product. The mother layer was evaporated and the residue was crystallized from DIPE. The precipitate was filtered off and dried, yielding 6.51 g of product The two fractions were put together and taken up in water and DCM, NaOH was added and the mixture was extracted. The organic layer was dried, filtered and the solvent evaporated, yielding 16.14 g (38 %) of (±)-1-[3,5-bis(trifluoromethyl)benzoyl]-2-(phenylmethyl)-4-piperidinone (intermediate 3; mp.102.5 °C).

B. Preparation of the compounds (I) 27 or 95

Example B.3

a) Titanium(IV)isopropoxide (13.2 g) was added to a mixture of intermediate 3 (17.16 g) and *N*-(2,6-dimethylphenyl)-1-piperazineacetamide (9.88 g) in DCM (20 ml). This mixture was stirred for 3 hours at RT. Sodium cyanoborohydride (2.52 g) in ethanol (20 ml) was added and the resulting reaction mixture was stirred overnight at RT. Water (10 ml) was added and the reaction mixture was extracted with DCM (800 ml). The organic layer was separated, dried, filtered and the solvent was evaporated. The residue was taken up into water and this mixture was extracted with DCM. The separated organic layer was dried, filtered, and the solvent evaporated. The residue was pre-purified by column chromatography over silica gel (eluent : $CH_2Cl_2/CH_3OH$ 97/3). The desired fractions were collected and the solvent was evaporated, giving 4 g of the trans-racemate. Resolution was obtained by purification over stationary phase Chiralcel OD (eluent: $CH_3OH$ 100%). Two desired *trans*-fraction groups were collected and their solvent was evaporated, yielding 1.75 g fraction 1 and 2 g fraction 2. Fraction 1 was dissolved in DCM, filtered and the filtrate was evaporated. The residue was dried, yielding 1.55 g (6 %) (-)-(A)-*trans*-4-[1-[3,5-bis(trifluoromethyl)benzoyl]-2-(phenylmethyl)-4-piperidinyl]-*N*-(2,6-dimethylphenyl)-1-piperazine acetamide (compound 26; mp. 97.4 ˚C; $[\alpha]_D^{20} = -5.81°$ (c =1 % in DMF)). Fraction 2 was dissolved in DCM, filtered and the filtrate was evaporated. The residue was dried, yielding 1.70 g (6 %) (+)-(B)-*trans*-4-[1-[3,5-bis(trifluoromethyl)benzoyl]-2-(phenylmethyl)-4-piperidinyl]-*N*-(2,6-dimethylphenyl)-1-piperazine acetamide (compound 27; mp. 96.8˚C; $[\alpha]_D^{20} = +5.71°$ (c = 1 % in DMF)).

b) Compound 27 was dissolved in warm 2-propanol and converted into the (L)-malic acid salt with a solution of (L)-malic acid in 2-propanol. The mixture was stirred for 2 hours and the precipitate was filtered off and dried, yielding (+)-(B)-*trans*-4-[1-[3,5-bis(trifluoromethyl)benzoyl]-2-(phenylmethyl)-4-piperidinyl] -*N*-(2,6-dimethylphenyl)-1-piperazine acetamide (L)-malic acid (1:1) (compound 95).

Table 2 list compounds 26 and 27 and 95.

Table 2

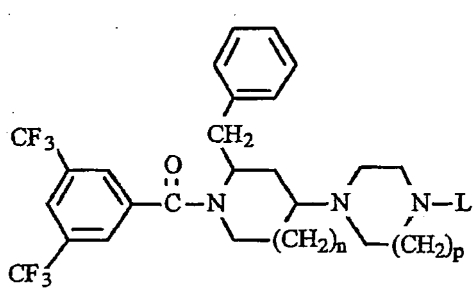

| Co. No. | Ex. | n | p | -L | Physical data (mp = melting point) |
|---|---|---|---|---|---|
| 26 | B.1 and B.3 | 1 | 1 | 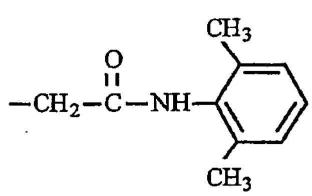 | (-)-(A)-*trans;* mp 97.4˚C; $[\alpha]_D^{20} = -5.81°$ (c = 1 % in DMF) |

(continued)

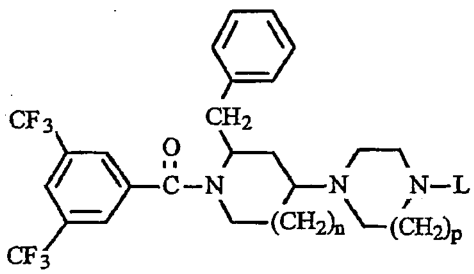

| Co. No. | Ex. | n | p | -L | Physical data (mp = melting point) |
|---|---|---|---|---|---|
| 27 | B.1 and B.3 | 1 | 1 | 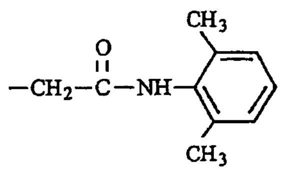 | (+)-(B)-*trans*; mp 96.8˚C; $[\alpha]_D^{20}$ = +5.71° (c=1% inDMF) |
| 95 | B4 | 1 | 1 | 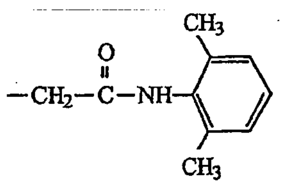 | (B)-*trans ;* (L)-malic acid (1: 1) |

C. Pharmacological examples

C.1 : $NK_1$-antagonism

The $NK_1$-antagonistic activity of the compounds in the Table 2 has been disclosed in WO 97/16440-A1 which is disclosed herein by reference.
Unless otherwise specified, all tests were performed with Compound 95 : ((+)-(B)-*trans*-4-[1-[3,5-bis(trifluoromethyl) benzoyl]-2-(phenylmethyl)-4-piperidinyl]-*N*-(2,6-dimethylphenyl)-1-piperazine acetamide, (L)-malic acid (1:1)).

C.2 : Inhibition of morphine-induced emesis in ferrets

In order to test whether a $NK_1$ antagonist was also able to overcome emesis when co-administered with a centrally acting opioid, ascending doses of Compound 95 were tested against different concentrations of morphine in non food-deprived ferrets after i.p. administration.
Based on preliminary data, doses of 0.4 and 0.8 mg/kg morphine were selected. Increasing the dose from 0.4 to 0.8 mg/kg morphine resulted in more animals with emesis and increased frequencies of periods of emesis, retches and vomiting (Table 5). Doses > 0.8 mg/kg morphine caused central side-effects.
At 0.4 mg/kg morphine, a submaximal dose which did not result in emesis in all animals tested, there was a non-significant tendency with Compound 95 to reduce the frequency of retches, vomiting and periods of emesis.
At 0.8 mg/kg morphine, a more robust drug-induced emesis was present. Doses of 10 and 40 mg/kg i.p. Compound 95 significantly reduced the number of retches. The number (frequency) of vomits diminished significantly in the presence of 10 mg/kg Compound 95 i.p. In terms of periods of emesis, activity was seen at doses ≥ 2.5 mg/kg Compound 95.
These results indicate that $NK_1$ antagonists can overcome opioid-induced emesis even when co-administered with the opioids.

Table 5 : Effects of increasing doses of Compound 95 on morphine-induced emesis in ferrets

Given are the mean (SEM) results of 8 ferrets/condition treated simultaneously i.p. with various doses of Compound 95 and morphine. Differences from vehicle controls were evaluated using the MWU test (two-tailed; *: $p < 0.05$; **: $p < 0.01$)

| | | Latency of onset (sec) | | Frequency | | | |
|---|---|---|---|---|---|---|---|
| **Challenge** | **Dose Compound 95 (mg/kg i.p.)** | **Retching** | **Vomiting** | **Retches** | **Vomiting** | **Periods of emesis** | **Duration of emesis (sec)** |
| Morphine 0.4 mg/kg i.p. *n* = 8 per group | Vehicle | **357.86± (90.99)** | **471.67± (123.98)** | **22.88+ (6.49)** | **2.63± (0.92)** | **4.38+(0.94)** | **6.39±(0.95)** |
| | 0.16 | 217.00± (50.62) | 290.80± (52.25) | 28.00± (10.70) | 2.50± (1.07) | 4.88± (1.59) | 7.37±(1.63) |
| | 0.63 | 247.17+ (43.87) | 263.6± (51.71) | 36.25± (13.97) | 3.25± (1.39) | 5.38± (2.03) | 8.90±(1.35) |
| | 2.5 | 313.75± (64.34) | 502.25± (234.81) | 17.88± (8.50) | 2.38± (1.18) | 3.13± (1.41) | 9.10±(2.16) |
| | 10 | 329.60+ (63.10) | 373.25+ (33.99) | 12.63+ (5.95) | 1.00+(0.50) | 2.63+(1.19) | 6.78+(1.21) |
| Morphine 0.8 mg/kg i.p. *n* = 8 per group | Vehicle | **196.25± (26.43)** | **218.00± (24.03)** | **45.25± (10.12)** | **5.25± (1.56)** | **9.50± (2.17)** | **8.41±(1.07)** |
| | 0.63 | 216.38+ (31.51) | 347.00± (30.44)* | 25.50± (6.18) | 1.50± (0.65) | 4.75± (1.26) | 9.05±(1.24) |
| | 2.5 | 251.57± (27.52) | 301.20± (54.78) | 22.75± (5.53) | 1.88± (0.79) | 3.50± (0.71)* | 6.20±(1.50) |
| | 10 | 353.29± (94.38) | 399.75± (122.43) | 15.25± (4.39)* | 0.88± (0.35)* | 3.50± (0.98)* | 5.20±(1.13) |
| | 40 | 275.50± (84.10) | 348.25± (74.57) | 9.63±(5.40) ** | 1.13± (0.61) | 3.00± (1.55)* | 5.10±(1.07) |

C.3 : Respiratory depression

In order to evaluate the effects of combinations of opioids with $NK_1$ antagonists on respiratory depression, experiments were performed in gerbils, guinea pigs and rats, measuring arterial blood gases, and especially opioid-induced $P_{CO_2}$ increases over time after drug treatment using a blood gas analyzer (ABL Radiometer Copenhagen).

On the day of testing, male gerbils were equipped with a catheter in the arteria femoralis. After full recovery from surgery and habituation to the test conditions, predrug baselines were taken. Only animals with $P_{CO_2}$ blood levels < 30 mmHg were included for drug testing. Drugs were injected s.c. and samples were taken at repeated intervals after treatment.

Opioids resulted in an increase in $P_{CO_2}$ values over time. With Compound 95, up to 10 mg/kg s.c., no effects were observed. Adding 10 mg/kg s.c. Compound 95 to the highest doses of fentanyl (0.63 mg/kg s.c.) and morphine (10 mg/kg s.c.), resulted in significant decreases in the opioid-induced $P_{CO_2}$ levels

These data indicate that Compound 95 can reduce part of the respiratory depression induced by opioids. Furthermore, a role of SP in the respiratory system is suggested in the literature. If the effects of Compound 95 reflect here an antagonism of the opioid-induced excitation, this can be beneficial because opioid-induced excitation is sometimes seen at overdosing.

In both guinea pigs and rats, opioids do not result in excitation and therefore the effects of Compound 95 on the opioid-induced $P_{CO_2}$ levels should be more directly related to effects in the respiratory system.

Guinea pigs were chronically catheterized with an intra-arterial line into the arteria carotis. In order to keep the lines patent, the animals were connected to chronic infusion pumps in their home cages and minimal amounts of heparine

in saline were administered daily. After full recovery of the animals and habituation to the chronic housing conditions, the animals were disconnected from the pumps and after s.c. drug treatment, samples were taken in their home cages at repeated time intervals.

Both fentanyl and morphine resulted in an increase in $P_{CO_2}$. With fentanyl, higher increases in $P_{CO_2}$ were generally measured. Compound 95, tested at doses ranging from 0.16 to 10 mg/kg s.c., did not affect $P_{CO_2}$ values. Adding Compound 95 to both fentanyl and morphine resulted in reductions in the opioid-induced $P_{CO_2}$ levels . The effects were more pronounced with fentanyl, probably due to the high doses tested.

Rats were acutely equipped with a catheter in the arteria femoralis. After full recovery from surgery and habituation to the test conditions, predrug baseline values were taken. Only animals with $P_{CO_2}$ blood levels < 35 mm Hg were included. Drugs were injected s.c. and samples were taken at repeated intervals after treatment.

As in the other species, the opioids fentanyl and morphine both resulted in increased levels of $Pco_2$ at the highest doses tested. Compound 95 at 40 mg/kg s.c. did not affect the $P_{CO_2}$ levels. Adding 40 mg/kg s.c. Compound 95 to the opioids resulted in some attenuation of the opioid-induced increases in $P_{CO_2}$, especially at the highest doses tested. Further analysis indicated that Compound 95 especially reduced the number of animals reaching high (>50 mm Hg) levels of $P_{CO_2}$.

Thus, the data in rats confirm the observations in guinea pigs and gerbils that the $NK_1$ antagonist Compound 95 can reduce the opioid-induced increases in $P_{CO_2}$, pointing to some functional role of $NK_1$ antagonists on the opioid-induced respiratory depression.

C.4 : Constipation : the castor oil test

In order to evaluate the constipatory effects of opioids in combination with $NK_1$ antagonists, a castor oil test was performed in gerbils and rats. In both species, and one hour after s.c. pretreatment with the test compounds, animals were challenged with 0.25 (gerbils) or 1 ml (rats) ricinus oil, and the number of animals showing diarrhea were measured every hour up to 8 hours after the challenge. Because, in control animals (treated with vehicle), >95 % revealed diarrhea within 4 (rats) and 8 h (gerbils), respectively, these time points were used to calculate $ED_{50}$s for opioid-induced constipation (opioid-induced inhibition of diarrhea).

All opioids tested (morphine, fentanyl, codeine and oxycodone in gerbils and morphine and fentanyl in rats) resulted in a dose-dependent inhibition of the ricinus oil -induced diarrhea (Table 6). Compound 95, at doses up to 40 mg/kg s.c., had minimal effects on constipation in both rats and gerbils.

Adding Compound 95 to the opioids did not alter the $ED_{50}$s of the opioids, neither in gerbils nor in rats (Table 6).

Table 6 : Effects of opioids and Compound 95 in the castor oil test in gerbils and rats.

| Given are the $ED_{50}$s (95% CL) values in mg/kg of the opioids to postpone the ricinus oil-induced diarrhea for 8 (gerbils) and 4 h. (rats) respectively. For each test condition n=5 were used. No differences between the $ED_{50}$s of the opioid and the respective combinations with Compound 95 were observed. | | | | | |
|---|---|---|---|---|---|
| | gerbils | | | rats | |
| Compound | vehicle | Compound 95 0.16 mg/kg s.c. | Compound 95 2.5 mg/kg s.c. | vehicle | Compound 95 40 mg/kg s.c. |
| Fentanyl | 0.080 (0.036-0.18) | 0.14 (0.062-0.31) | 0.020 (0.009-0.044) | 0.11 (0.06-0.19) | 0.11 (0.05-024) |
| Morphine | 0.72 (0.29-1.89) | 1.66 (0.50-5.48) | 0.32 (0.096-1.05) | 2.19 (1.19-4.01) | 0.95 (0.52-1.75) |
| Oxycodone | 5.02 (1.92-13.1) | 11.51 (6.28-21.11) | 5.02 (2.84-11.27) | - | - |
| Codeine | 1.67 (0.64-4.35) | 1.67 (0.74-3.74) | 2.20 (0.38-4.93) | - | - |

C.5 : Gastrointestinal transit : the charcoal test

In order to evaluate the effects of adding an $NK_1$ antagonist to the effects of opioids on gastrointestinal transit, a charcoal test was performed in male gerbils and rats. For testing, the animals were pretreated s.c. with the test compound, lh prior to the oral administration of 1 ml (gerbil) or 2 ml (rat) charcoal (10 % in 5% Arabic gum) per 100 g body weight. After 20 min (gerbil) and 30 min (rat), the animals were sacrificed, the charcoal propulsion was measured

throughout the small intestine and the peristalsis ratio was calculated. This is the ratio between the distance attained by the charcoal in the small intestines (as measured from the end of the stomach (pylorus)) and the total length of the small intestines (up to the beginning of the caecum). Because, in control rats (n = 30) and gerbils (n = 155), a peristalsis ratio <60 % was never observed, this level was used as an all or nothing criterion to calculate GI tract inhibition.

In gerbils, all opioids resulted in a dose-dependent inhibition of the charcoal propulsion while Compound 95 was inactive. Adding Compound 95 to the opioids did not affect the outcome of the opioids (Table 7).

In rats, comparable results were obtained with morphine and Compound 95. With fentanyl, tested up to 0.16 mg/kg s.c., no inhibition of the charcoal transit was observed, also when combined with 40 mg/kg s.c.Compound 95 (Table 7).

Table 7 : Effects of opioids and Compound 95 in the charcoal test in gerbils and rats.

| Given are the $ED_{50}$s (95% CL) in mg/kg of the opioids for an inhibition of charcoal propulsion (<60 %). No differences between the $ED_{50}$s of the opioids and the respective combinations were observed | | | | | |
|---|---|---|---|---|---|
| | gerbils | | | rats | |
| | vehicle | Compound 95 0.16 mg/kg s.c. | Compound 95 2.5 mg/kg s c | vehicle | Compound 95 40 mg/kg s.c. |
| Fentanyl | 0.032 (0.022-0.049) | 0.043 (0.032-0.058) | 0.057 (0.042-0.077) | > 0.16 | >0.16 |
| Morphine | 0.043 (0.032-0.058) | 0.037 (0.028-0.051) | 0.057 (0.042-0.077) | 1.66 (0.74-3.72) | 3.81 (2.08-6.98) |
| Oxycodone | 1.26 (0.82-1.93) | 2.19 (0.97-4.90) | 1.66 (0.63-4.32) | - | - |
| Codeine | 0.9 (0.42-2.14) | 1.26 (0.48-3.28) | 0.41 (0.14-0.23) | - | - |

Taken together as indices for GI activity, the castor oil and the charcoal test results in both rats and gerbils confirm that opioids have constipatory effects, which were not potentiated by Compound 95.

C.6 : Tolerance in a chronic neuropathic pain model

In order to evaluate the effects of adding a $NK_1$ antagonist on the opioid-induced tolerance over time in a chronic pain model, experiments were performed in gerbils with a chronic constrictive injury (CCI) using the acetone spray test. In CCI gerbils, acetone is demonstrated to produce an increased pain reactivity (cold/chemical hyperalgesia). This pain reactivity can be acutely antagonized by opioids, but not by $NK_1$ antagonists.

To test whether Compound 95 could prevent the loss of efficacy of morphine over time in this model, CCI gerbils were tested over an 11 days period receiving twice daily i.p. injections of either vehicle, 2.5 mg/kg morphine, 2.5 mg/kg Compound 95 or the combination of 2.5 mg/kg morphine with 2.5 mg/kg Compound 95.

As was seen, morphine acutely reduced the acetone-induced lifting behaviour but reductions of activity became apparent from the second day onwards. At day 7, no differences with the controls were present anymore. With Compound 95, limited activity was present during some days of the experiment. The combination of Compound 95 with morphine resulted in an initial activity at the first day similar to morphine alone, and as opposed to morphine, the activity remained present over the entire testing period.

These data indicate that, under certain testing conditions, an $NK_1$ antagonist can overcome the loss of efficacy of an opioid over time, pointing to the prevention of the development of tolerance to the functional effects of these drugs.

C.7 : Discriminative stimulus properties of opioids

In order to evaluate whether a $NK_1$ antagonist could interfere with the abuse potential, and especially the central narcotic properties of opioids, the effects of Compound 95 on the discriminative stimulus properties of fentanyl were studied in rats trained to discriminate 0.04 mg/kg s.c. fentanyl from saline in a two lever food reinforced test procedure.

The $ED_{50}$ (95 % CL) values of fentanyl for stimulus generalization in fentanyl trained rats injected s.c. 30 and 60 min prior to testing were 0.018 (0.012-0.028) and 0.021 (0.016-0.029) mg/kg, respectively.

Compound 95 at doses ranging from 0.63 to 40 mg/kg s.c. did not show any generalization to nor any antagonism

of the fentanyl cue.

Pretreatment with 10 or 40 mg/kg Compound 95 did not affect the stimulus generalization of fentanyl in the fentanyl trained rats. No shifts in the dose response curves of fentanyl were observed, neither was there a change in the $ED_{50}$ values for stimulus generalization (Table 8).

Table 8 : Effects of Compound 95 on the cueing properties of fentanyl in rats.

| Given in mg/kg are the $ED_{50}$s (95 % CL) of fentanyl for stimulus generalization after pretreatment with various doses of Compound 95 in rats trained to discriminate 0.04 mg/kg fentanyl from saline in a two lever food reinforced drug discrimination test procedure | |
|---|---|
| Pre-treatment (s.c., 60 min prior to test) | $ED_{50}$(mg/kg) |
| Vehicle | 0.0193 (0.014-0.027) |
| 10 mg/kg Compound 95 | 0.016 (0.011-0.021) |
| 40 mg/kg Compound 95 | 0.013 (0.0085-0.019) |

These results clearly indicate that, in rats, Compound 95 does not interfere with the discriminative stimulus properties of the opioid fentanyl.

### Claims

1. A pharmaceutical composition comprising a pharmaceutically acceptable carrier and, as active ingredients, an opioid analgesic and a therapeutically effective amount of compound:

   *trans*-4-[1-[3,5-bis(trifluoromethyl)benzoyl]-2-(phenylmethyl)-4-piperidinyl]-*N*-(2,6-dimethylphenyl)-1-piperazine acetamide ;

   the pharmaceutically acceptable acid or base addition salts thereof, and the *N*-oxide form thereof

2. A pharmaceutical composition according to claim 1, wherein the therapeutically effective compound is present as (+)-*trans*-4-[1-[3,5-bis(trifluoromethyl)benzoyl]-2-(phenylmethyl)-4-piperidinyl]-*N*-(2,6-dimethylphenyl)-1-piperazine acetamide.

3. A pharmaceutical composition according to any one of claims 1 to 2, wherein the salt is (L)-malic acid (1:1).

4. A pharmaceutical composition according to any one of claims 1 to 3, **characterized in that** it is formulated for simultaneous, separate, or sequential use.

5. A pharmaceutical composition according to any one of claims 1 to 4, **characterized in that** the opioid analgesic is one or more compounds selected from the group of alfentanil, buprenorphine, butorphanol, carfentanyl, codeine, diacetylmorphine, dihydrocodeine, fentanyl, hydrocodone, hydromorphone, levorphanol, lofentanyl, meperidine, methadone, morphine, nalbuphine, oxycodone, oxymorphone, pentazocine, propoxyphene, remifentanyl and sufentanyl; and derivatives and pharmaceutical acceptable salts thereof.

6. A pharmaceutical composition according to claim 5, **characterized in that** the opioid analgesic is one or more compounds selected from the group of oxycodone, codeine, morphine, fentanyl, buprenorphine, hydrocodone, hydromorphone, and pharmaceutical acceptable salts and derivatives thereof.

7. A pharmaceutical composition according to any one of claims 1 to 6, **characterized in that** it is in a form suitable to be orally administered.

8. The use of a pharmaceutical composition according to any one of claims 1 to 7, for the manufacture of a medicament for the prevention and/or treatment of pain and/or nociception.

9. The use of a pharmaceutical composition according to any one of claims 1 to 7, for the manufacture of a medicament

for the prevention and/or treatment of acute and chronic pain, more in particular in inflammatory, post-operative, emergency room (ER), breakthrough, neuropathic, and cancer pain treatments.

**10.** The use of a pharmaceutical composition according to any one of claims 1 to 7, for the manufacture of a medicament for the prevention and/or treatment of emesis in opioid-based treatments of pain.

**11.** The use of a pharmaceutical composition according to any one of claims 1 to 7, for the manufacture of a medicament for the prevention and/or treatment of nausea and vomiting in opioid-based treatments of pain.

**Patentansprüche**

**1.** Pharmazeutische Zusammensetzung, enthaltend einen pharmazeutisch unbedenklichen Träger und, als Wirkstoffe, ein Opioidanalgetikum und eine therapeutisch wirksame Menge der Verbindung:

trans-4-[1-[3,5-Bis(trifluormethyl)benzoyl]-2-(phenylmethyl)-4-piperidinyl]-*N*-(2,6- dimethylphenyl)- 1- piperazinacetamid;

oder eines pharmazeutisch unbedenklichen Säure- oder Basenadditionssalzes davon oder der N-Oxidform davon.

**2.** Pharmazeutische Zusammensetzung nach Anspruch 1, wobei die pharmazeutisch wirksame Verbindung als (+)-*trans*-4-[1-[3,5-Bis(trifluormethyl)benzoyl]-2-(phenylmethyl)-4-piperidinyl]-*N*-(2,6-dimethylphenyl)-1-piperazinacetamid vorliegt.

**3.** Pharmazeutische Zusammensetzung nach Anspruch 1 oder 2, wobei es sich bei dem Salz um (L)-Äpfelsäure (1: 1) handelt.

**4.** Pharmazeutische Zusammensetzung nach einem der Ansprüche 1 bis 3, **dadurch gekennzeichnet, daß** sie für die gleichzeitige, getrennte oder aufeinanderfolgende Anwendung formuliert ist.

**5.** Pharmazeutische Zusammensetzung nach einem der Ansprüche 1 bis 4, **dadurch gekennzeichnet, daß** es sich bei dem Opioidanalgetikum um eine oder mehrere Verbindungen ausgewählt aus der Gruppe Alfentanil, Buprenorphin, Butorphanol, Carfentanyl, Codein, Diacetylmorphin, Dihydrocodein, Fentanyl, Hydrocodon, Hydromorphon, Levorphanol, Lofentanyl, Meperidin, Methadon, Morphin, Nalbuphin, Oxycodon, Oxymorphon, Pentazocin, Propoxyphen, Remifentanyl und Sufentanyl und den Derivaten und pharmazeutisch unbedenklichen Salzen davon handeln.

**6.** Pharmazeutische Zusammensetzung nach Anspruch 5, **dadurch gekennzeichnet, daß** es sich bei dem Opioidanalgetikum um eine oder mehrere Verbindungen ausgewählt aus der Gruppe Oxycodon, Codein, Morphin, Fentanyl, Buprenorphin, Hydrocodon, Hydromorphon und pharmazeutisch unbedenklichen Salzen und Derivaten davon handeln.

**7.** Pharmazeutische Zusammensetzung nach einem der Ansprüche 1 bis 6, **dadurch gekennzeichnet, daß** sie in einer für die orale Verabreichung geeigneten Form vorliegt.

**8.** Verwendung einer pharmazeutischen Zusammensetzung nach einem der Ansprüche 1 bis 7 zur Herstellung eines Medikaments zur Prävention und/oder Behandlung von Schmerzen und/oder Nozizeption.

**9.** Verwendung einer pharmazeutischen Zusammensetzung nach einem der Ansprüche 1 bis 7 zur Herstellung eines Medikaments zur Prävention und/oder Behandlung von akuten und chronischen Schmerzen, insbesondere der Behandlung von entzündlichen Schmerzen, postoperativen Schmerzen, Notfallschmerzen, Durchbruchschmerzen, neuropathischen Schmerzen und Krebsschmerzen.

**10.** Verwendung einer pharmazeutischen Zusammensetzung nach einem der Ansprüche 1 bis 7 zur Herstellung eines Medikaments zur Prävention und/oder Behandlung von Emesis bei der auf Opioiden basierenden Behandlung von Schmerzen.

**11.** Verwendung einer pharmazeutischen Zusammensetzung nach einem der Ansprüche 1 bis 7 zur Herstellung eines

Medikaments zur Prävention und/oder Behandlung von Übelkeit und Erbrechen bei der auf Opioiden basierenden Behandlung von Schmerzen.

## Revendications

1. Composition pharmaceutique comprenant un véhicule pharmaceutiquement acceptable et, en tant qu'ingrédients actifs, un analgésique opioïde et une quantité thérapeutiquement efficace du composé :

   trans- 4-[1-[3,5- bis (trifluorométhyl) benzoyl]- 2-(phénylméthyl)- 4- pipéridinyl]-N-(2,6- diméthylphényl)- 1-pipérazinacétamide ;

   les sels d'addition d'acide ou de base pharmaceutiquement acceptables de celui-ci, et la forme *N*-oxyde de celui-ci.

2. Composition pharmaceutique selon la revendication 1, dans laquelle le composé thérapeutiquement efficace est présent sous forme de (+)-*trans*-4-[1-[3,5-bis(trifluorométhyl)benzoyl]-2-(phénylméthyl)-4-pipéridinyl]-N-(2,6-diméthylphényl)-1-pipérazinacétamide.

3. Composition pharmaceutique selon l'une quelconque des revendications 1 à 2, dans laquelle le sel est l'acide (L)-malique (1:1).

4. Composition pharmaceutique selon l'une quelconque des revendications 1 à 3, **caractérisée en ce qu'**elle est formulée pour utilisation simultanée, séparée ou séquentielle.

5. Composition pharmaceutique selon l'une quelconque des revendications 1 à 4, **caractérisée en ce que** l'analgésique opioïde est un ou plusieurs composés choisis dans le groupe constitué de l'alfentanil, la buprénorphine, le butorphanol, le carfentanyl, la codéine, la diacétylmorphine, la dihydrocodéine, le fentanyl, l'hydrocodone, l'hydromorphone, le lévorphanol, le lofentanyl, la mépéridine, la méthadone, la morphine, la nalbuphine, l'oxycodone, l'oxymorphone, la pentazocine, le propoxyphène, le rémifentanyl et le sufentanyl ; et les dérivés et sels pharmaceutiquement acceptables de ceux-ci.

6. Composition pharmaceutique selon la revendication 5, **caractérisée en ce que** l'analgésique opioïde est un ou plusieurs composés choisis dans le groupe constitué de l'oxycodone, la codéine, la morphine, le fentanyl, la buprénorphine, l'hydrocodone, l'hydromorphone, et les sels et dérivés pharmaceutiquement acceptables de ceux-ci.

7. Composition pharmaceutique selon l'une quelconque des revendications 1 à 6, **caractérisée en ce qu'**elle est sous une forme adaptée pour être administrée par voie orale.

8. Utilisation d'une composition pharmaceutique selon l'une quelconque des revendications 1 à 7, pour la fabrication d'un médicament pour la prévention et/ou le traitement de la douleur et/ou de la nociception.

9. Utilisation d'une composition pharmaceutique selon 1'une quelconque des revendications 1 à 7, pour la fabrication d'un médicament pour la prévention et/ou le traitement de la douleur aiguë et chronique, plus particulièrement dans des traitements de douleur inflammatoire, postopératoire, en service d'urgences, aiguë, neuropathique et cancéreuse.

10. Utilisation d'une composition pharmaceutique selon l'une quelconque des revendications 1 à 7, pour la fabrication d'un médicament pour la prévention et/ou le traitement des vomissements dans les traitements à base d'opioïdes de la douleur.

11. Utilisation d'une composition pharmaceutique selon l'une quelconque des revendications 1 à 7, pour la fabrication d'un médicament pour la prévention et/ou le traitement de la nausée et des vomissements dans les traitements à base d'opioïdes de la douleur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9716440 A1
- WO 9620009 A, Merck
- WO 9725988 A, Eli Lilly
- GB 2287 A
- WO 0130371 A
- WO 0232867 A
- EP 0532456 A
- EP 532456 A


### Non-patent literature cited in the description


- **BERTRAND ; GEPPETTI.** *Trends Pharmacol. Sci.,* 1996, vol. 17, 255-259
- **LUNDBERG.** *Can. J. Physiol. Pharmacol.,* 1995, vol. 73, 908-914
- **MAGGI.** *Gen. Pharmacol,* 1995, vol. 26, 911-944
- **REGOLI et al.** *Pharmacal. Rev.,* 1994, vol. 46
- **MAGGI ; SCHWARTZ.** *Trends Pharmacol. Sci.,* 1997, vol. 18, 351-355
- **MAGGI.** *Gen. Pharmacol.,* 1995, vol. 26 (911-944
- **REGOLI et al.** *Pharmacal. Rev.,* 1994, vol. 46 (4), 551-599
- **LONGMORE J. et al.** *DN&P,* 1995, vol. 8 (1), 5-23
- **BOUNTRA et al.** *European Journal of Pharmacology,* 1993, vol. 249
- **TATTERSALL et al.** *Neuropharmacology,* 1996, vol. 33 (2), 259-260
- *Textbook of Organic Medicinal and Pharmaceutical Chemistry,* 1977, 70-75
- **STELLA, V. J. et al.** Prodrugs. *Drug Delivery Systems,* 1985, 112-176
- **DRUGS.** *Drugs,* 1985, vol. 29, 455-473
- **WATSON et al.** *Br. J. Pharmacol.,* 1995, vol. 115, 84-94